# EUROPEAN PATENT SPECIFICATION

(11) **EP 2 548 584 B1**
(45) Date of publication and mention of the grant of the patent: **18.03.2015**
(21) Application number: 12176744.6
(22) Date of filing: 17.07.2012
(51) Int. Cl.: A61L 2/18, A61L 2/22

(54) **Sanitization device for trolleys**
Desinfektionsvorrichtung für Rollwagen
Dispositif de désinfection pour chariots

(30) Priority: 18.07.2011 IT AN20110099
(43) Date of publication of application: 23.01.2013
(73) Proprietor: Perinetti, Franco, 65010 Spoltore (PE) (IT)
(72) Inventor: Perinetti, Franco, 65010 Spoltore (PE) (IT)
(74) Representative: Baldi, Claudio

(56) References cited:
- WO-A1-2011/076476
- WO-A2-2007/139831
- US-A- 3 698 029
- US-A1- 2005 121 057
- US-A1- 2005 217 701
- US-A1- 2006 011 220
- US-A1- 2008 029 133

## Description

The present patent application for industrial invention relates to a sanitization device for trolleys, in particular shopping trolleys, such as the ones that are commonly used in supermarkets, department stores or the like.

As it is known, the problem related with the large diffusion of harmful viruses and bacteria transmitted from man to man has recently acquired growing importance because they are able to spread flu epidemics and other diseases, including severe ones.

In view of the above, the use of wipes soaked with disinfectants or small disinfectant bottles has become largely popular, being applied by users on the objects touched by them in order to avoid the contraction of viruses and bacteria.

However, users must always carry said sanitizing wipes and dispose of them after use in waste containers that are not always within reach.

These inconveniences are especially felt when an object is manipulated by a large number of people, thus making the diffusion of germs, bacterias and viruses extremely probable.

Such is the case, for example, of shopping trolleys, like the ones that are normally used in supermarkets or department stores, when a very high number of people use the same trolley and come in contact with its push bar.

In view of the above, the push bar is a great vehicle of diffusion of germs, bacteria and viruses. Such a situation is worsened considering that, after grabbing the push bar, users may use their hands to hold fruit and vegetable, contaminating them.

Users may decide to use a wipe soaked with disinfectant to clean the push bar of the trolley or wear latex gloves. However, such remedies are not very practical and are rarely applied, with the consequence that the diffusion of germs, bacteria and viruses is often high.

US2006/0011220 discloses a shopping cart wash tunnel.

US2008/0029133 discloses a portable cleaning system for shopping carts.

WO2011 /076476 discloses a cleaning device for disinfecting shopping trolley handles.

US2005/0217701 discloses a shopping cart sanitizing system acting on all the surface of the shopping cart.

US 3 698 029 discloses an apparatus for automatically washing nestable shopping carts.

WO2007/139831 discloses a sanitizing system for shopping carts comprising an enclosure through which the carts may be transported.

US2005/121057 discloses a system for disinfecting shopping carts.

The purpose of the present invention is to provide a sanitization device for trolleys, in particular shopping trolleys, such as the ones that are normally used in supermarkets, department stores, or the like, which is practically and rapid to use and can be easily installed in points of sales that use said trolleys to sanitize the push bar.

The purpose of the present invention is a sanitization device for trolleys, in particular shopping trolleys, such as the ones that are normally used in supermarkets, department stores or the like, which is realized in accordance with the first claim herein attached.

Therefore, the user can position the push bar of the trolley under the dispenser and actuate (according to various operating modes) the distribution of said sanitizing product on the push bar, which can be safely held by the user without the risk of contacting germs, viruses or bacteria.

The sanitization device comprises a receiver-transmitter coupled with a transponder located on said trolley, in order to activate a dispensing cycle of said sanitizing product when said receiver-transmitter detects the presence of the transponder of the trolley.

Advantageous characteristics of the present invention are the object of the attached dependent claims.

For explanatory reasons, the description of the device according to the present invention continues with reference to attached drawings, which only have illustrative, non-limiting value, wherein:
Fig. 1 is a perspective view of the device of the present invention;
Fig. 2 is a view of the device of Fig. 1 with a trolley in ready position;
Fig. 3 is a view of the device of the invention in a working, or sanitization condition of the push bar of the trolley;
Fig. 4 is a cross-sectional view of a dispenser of the device of the invention;
Fig. 5 is a diagrammatic axial view of a second embodiment of the sanitization device of the invention;
Fig. 6 is a cross-sectional view along section plane VI-VI of Fig. 5;
Fig. 7 is a partially interrupted perspective view showing a portion of the sanitization device of the invention.

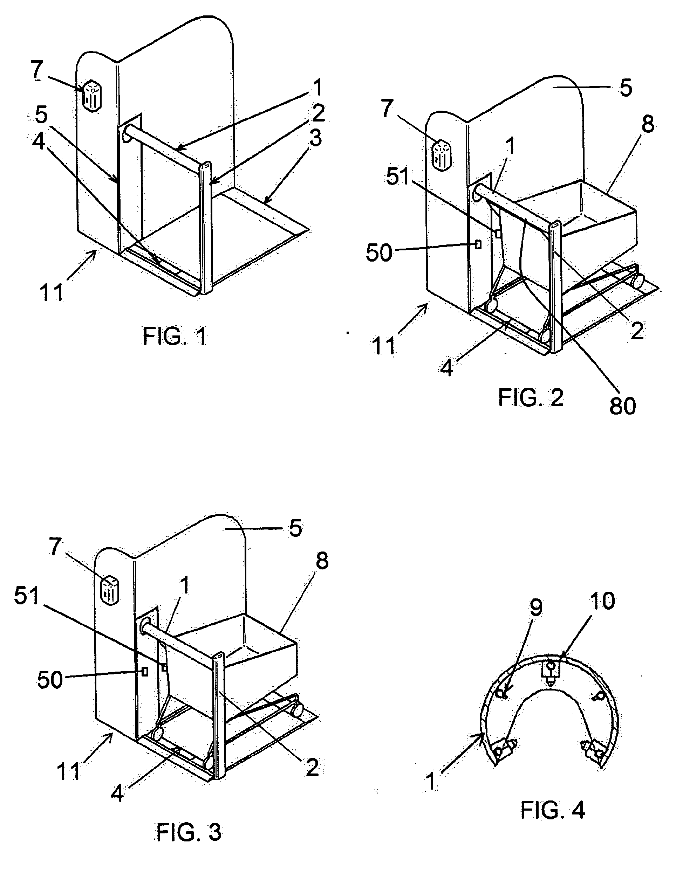

Referring to Figs. 1 - 3, a sanitization device (11) for trolleys (8) is disclosed, in particular for shopping trolleys, of the type comprising a trolley body and a push bar (80), such as trolleys of supermarkets, department stores or the like.

The device (11) of the present invention comprises a tank of sanitizing product, a dispenser (1) of said sanitizing product and positioning means (4) of the shopping trolley with respect to said dispenser (1).

The dispenser (1) is operatively connected to said tank of sanitizing product, eventually by means of pumps or the like, suitable for conveying the sanitizing product to said dispenser (1).

The positioning means (4) are made in such manner to position the push bar of the trolley (8) in correspondence of an active area of said dispenser (1) to sanitize it.

An "active area" of the dispenser (1) is an area where the dispenser performs its dispensing function; for example, if the dispenser (1) is provided with nozzles, the active area is the area in front of the nozzles; instead, if the dispenser is provided with microholes for dispensing the sanitizing product, the active area is the area in front of the microholes.

A sanitizing product is a product, either a disinfectant or not, able to reduce or completely eliminate the quantity of existing viruses, microbes, bacteria or germs.

In the example shown for illustrative, non-limiting purposes, the trolley comprises front and back wheels and the positioning means specifically comprise a concave guide (4) adapted to engage said back wheels of said trolley (8) for positioning the push bar correctly with respect to the dispenser (1). As shown in Fig. 2, when the back wheels of the trolley (8) are situated in the guide (4), the push bar (80) of the trolley is in a position that corresponds to the active area of the dispenser (1).

As specifically shown in Fig. 4, the dispenser (1) comprises a dispenser sleeve mainly extending in a direction; in particular, the dispenser sleeve has length equal or higher than the length normally given to push bars of trolleys (8) that can generally change according to the type and size of the trolley (8).

The dispenser sleeve is provided with dispensing nozzles (10) of the sanitizing product, which are preferably distributed uniformly along its length so that the sanitizing action affects the entire push bar of the trolley uniformly.

Preferably, the sleeve has a C-shaped cross-section, with opening facing downwards, towards the push bar of the trolley (8). The nozzles (10) are disposed internally to the C-shaped profile of the sleeve, viz. in its concavity, so that the active area mainly affects this portion of space.

According to the embodiment shown in the attached figures, the device (11) comprises actuation means of the dispenser (1). In view of the above, the dispenser (1) can move from a first waiting position (shown in Fig. 2) wherein the dispenser (1) is distal with respect to said push bar (80) of the trolley, with back wheels situated in the guide (4), to a second working, or sanitizing position (shown in Fig. 3), wherein the dispenser (1) embraces said push bar (80) of the trolley.

Optionally, the dispenser (11) comprises a platform (3) coupled with the guide (4); if said platform (3) is not present, the guide (4) rests directly on the ground.

In particular, the movement of the dispenser (1) from the ready position and the sanitization position is a linear movement along a basically vertical plane, so that the push bar (80) of the trolley is housed in the cavity of the C-shaped sleeve, in the active area of the dispenser (1), in such manner to close it and limit the distribution of the sanitizing product in the air during the operation of the device (11).

In order to automate the operation of the device (11), the dispenser (1) preferably comprises at least one obstacle sensor adapted to detect objects contained in a movement space of said dispenser (1).

In particular, the dispenser (1) can be actuated (both during movement and dispensing action) only in correspondence of a detection of said sensor excluding the presence of obstacles between the dispenser (1) and the push bar (80) of the trolley (8).

In view of the above, the safety of the user is guaranteed when involuntarily leaving his hands in the movement area of the dispenser (1).

According to a preferred embodiment, said obstacle sensor is provided with at least one safety photocell situated under the dispenser (1) and facing downwards.

Advantageously, when the sanitizing product is a liquid, the dispenser (11) also performs a drying action of the push bar; to that end, it preferably comprises a compressed air source and the dispenser (1) comprises dispensing nozzles (9) of compresses air operatively connected to said compressed air source and facing towards the push bar of said trolley (8).

In view of the above, after spraying the sanitizing product on the push bar, the push bar is air-dried, allowing the user to hold the push bar without coming into direct contact with the sanitizing product.

In general terms, the compressed air source is a pressurized air cylinder and/or a compressor.

Still with a view of providing an automatic intuitive operation of the device (11), it also comprises actuation means (not shown) of the guide (4), adapted to move it at least along one vertical direction.

So, when the sanitization of the push bar (80) is completed, the actuation means of the guide (4), will raise and/or tilt the guide (4), thus freeing the back wheels (8) to indicate that the sanitization of the push bar (80) is completed, and informing the user that the trolley (8) can be used.

According to the embodiment illustrated in the attached figures, the device (11) also comprises a housing case (5) coupled with said dispenser (1) and adapted to contain the functional parts of the device.

In particular, in the example, the case (5) contains at least the tank of sanitizing product; the compressed air source, the actuation means of the dispenser (1), and is coupled with the positioning means (4).

According to other embodiments, the case (5) contains only one part or a combination of said parts, according to the specific requirements.

According to the preferred embodiment illustrated in the attached figures, in order to reinforce the structure of the device (11), it also comprises a support column (2) for the dispenser (1). Therefore, the dispenser (1) is comprised and supported by the case (5) on one side and by the column (2) on the other side, thus preventing it from projecting from the case (5).

Finally, according to an optional, advantageous improvement, the case (5) also comprises a manual distributor (7) of sanitizing product, adapted to be actuated by the user to dispense a dose of sanitizing product for his hands.

The device (11) is provided with all functional elements adapted to guarantee operation, such as sensors, motors, buttons, indicator lights or acoustic signals.

As it can be understood from the foregoing description, a preferred mode is described hereinafter in detail.

The user extracts the trolley (8) from a row of trolleys and enters the supermarket to go shopping and wants to disinfect the handle of the trolley. Therefore he takes the trolley to the device of the invention and performs the following operations:
a. he pushes the trolley (8) under the dispensing sleeve (1) of the disinfecting product between the case (5) and the support column (2) until the back wheels of the trolley (8) fall into the guide (4);
b. an acoustic signal possibly informs the user that the disinfection operation will begin;
c. if the obstacle sensors do not detect any interposed objects, the dispensing sleeve (1) is lowered until it completely covers the push bar (80) of the trolley (8);
d. jets of disinfecting product are sprayed, nebulized or in general dispensed on the push bar of the trolley;
e. after disinfecting the push bar of the trolley, the user must wait until the push bar is dried, either naturally and/or by means of compressed air jets delivered by the dispenser (1);
f. an acoustic signal possibly informs the user that the operation has been completed and the dispensing sleeve (1) is raised until it releases the push bar of the trolley in order to push the trolley forward towards the exit.
g. The wheels of the trolley are raised from the positioning guide (4) by means of a mechanism provided in the guide and the trolley (8) can slide forward by a few centimeters, in order to allow the user to easily hold the push bar of the trolley and leave the sanitizing station.
h. During the sanitation the user can disinfect his hands by means of the distributor (7) provided on the wall of the case (5).

A receiver-transmitter (50) coupled with a transponder (51) mounted on the trolley is provided on the case (5) of the sanitizing device. The transponder (51) can be of passive RFID type in order to avoid the use of batteries. The receiver-transmitter (50) is set in such manner to recognize the transponder (51) of the trolley only when the trolley is positioned in the proximity of the dispenser (1), for example with the back wheels of the trolley in the guide (4).

The receiver-transmitter (50) enables the distribution of the sanitizing product by the dispenser (1) only when the receiver-transmitter (50) detects the transponder (51) of the trolley. In this way, the sanitizing product is not dispensed to foreign objects or trolleys that are not enabled by the transponder (51).

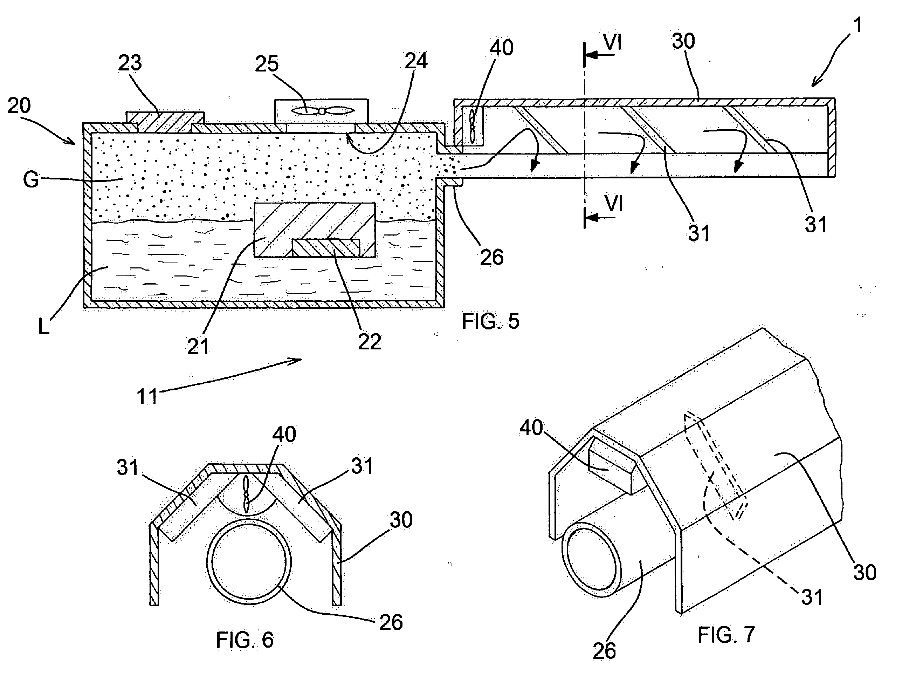

Figs. 5-6 illustrate a second embodiment of a sanitizing device (11).

The device (11) comprises a tank (20) containing sanitizing liquid (L) and a float (21) containing a nebulizer (22), preferably an ultrasound nebulizer. In view of the above, the nebulizer (22) is immersed in the liquid (L) in the proximity of the free surface of the liquid, allowing for the formation of nebulized steam (G) above the liquid (L) in the upper part of the tank (20).

The tank (20) has an inlet for the liquid closed by a removable cap (23). An opening (24) is provided in the upper part of the tank (20), in communication with a fan (25) to push the nebulized steam (G) towards the outlet (26) of the tank (20). The outlet (26) is coupled with the dispenser (1) to dispense nebulized steam.

Advantageously, the dispenser (1) comprises a sleeve (30) having a cross-section substantially semicircular or semi-octagonal in shape.

Inside the sleeve (30) a plurality of baffle fins (31) is disposed, projecting downwards in such manner that the nebulized gas (G) pushed by the fan (25) into the sleeve (30) is directed downwards above the push bar (80) of the trolley. The baffle fins (31) are suitably tilted with respect to the axis of the sleeve, in such manner to favor the forward movement of the nebulized gas.

Inside the sleeve (30), above the outlet (26), a hot air dryer (40) is provided, comprising for example electrical heating elements and a fan.

When the dispensing cycle of the nebulized gas of sanitizing product is completed, a drying cycle is started, when the dryer (40) sends a hot air jet inside the sleeve (30) to dry the sanitizing product that is left above the push bar (80) of the trolley.

## Claims

1. A sanitization device (11) for trolleys (8), said trolley (8) comprising a trolley body and a push bar (80),
said sanitization device (11) comprises:
- a tank (20) containing a sanitizing product (L),
- a dispenser (1) connected to said tank (20) for dispensing the sanitizing product,
- positioning means (4) for positioning said shopping trolley (8) with respect to said dispenser (1); said positioning means (4) being adapted to position said push bar (80) of the trolley in correspondence of an active area of said dispenser (1) to sanitize said push bar
**characterized in that**
said sanitization device (11) comprises a receiver-transmitter (50) coupled with a transponder (51) located on said trolley (8), in order to activate a dispensing cycle of said sanitizing product when said receiver-transmitter (50) detects the presence of the transponder (51) of the trolley.

2. A device (11) as claimed in claim 1, wherein said trolley comprises front and back wheels and said positioning means (4) comprise a concave guide (4) adapted to engage said back wheels of said trolley (8).

3. A device (11) as claimed in claim 1 or 2, wherein said dispenser (1) comprises a dispenser sleeve extending for the length of the push bar (80) of the trolley, said dispensing sleeve provided with dispensing nozzles (10) of said sanitizing product distributed along said dispenser sleeve.

4. A device (11) as claimed in claim 1, 2 or 3, comprising actuation means of said dispenser (1) suitable for moving said dispenser (1) from a first waiting position, wherein the dispenser is distal with respect to said push bar (80) of the trolley, and a second working position wherein the dispenser (1) embraces said push bar (80) of the trolley (8).

5. A device (11) as claimed in the preceding claim, wherein said dispenser (1) comprises at least one obstacle sensor adapted to detect objects contained in a movement space of said dispenser and prevent said dispenser (1) from being actuated when said sensor detects an obstacle.

6. A device (11) as claimed in any one of the preceding claims, comprising a compressed air source and wherein said dispenser (1) comprises dispensing nozzles (9) of compressed air connected to said compressed air source to dispense air towards said push bar (80) of the trolley (8).

7. A device (11) as claimed in any one of claims 2 to 6, comprising actuation means of said guide (4) to determine a movement of said guide in at least a vertical direction.

8. A device (11) as claimed in one or more of the preceding claims, comprising a housing case (5) coupled with said dispenser (1) and adapted to contain at least one between: said tank of sanitizing product, said compressed air source, said actuation means of said dispenser (1), and wherein said case (5) is coupled at least with said positioning means (4).

9. A device (11) as claimed in the preceding claim, wherein said case (5) comprises a manual distributor (7) of sanitizing product.

10. A device (11) as claimed in any one of the preceding claims, **characterized in that** said tank (20) comprises a nebulizer (22) to nebulize said sanitizing product (L) and a fan (25) to push said nebulized sanitizing product (G) toward said dispenser (1).

11. A device (11) as claimed in claim 10, **characterized in that** said sanitizing product is a liquid (L) and said nebulizer (22) is disposed in a float (21) floating on said liquid (L).

12. A device (11) as claimed in claim 10 or 11, **characterized in that** said dispenser comprises a sleeve (30) having a cross section semicircular or semi-octagonal in shape and baffle fins (31) protruding below into said sleeve (30) to baffle the nebulized gas (G) towards said push bar (80) of the trolley.

13. A device (11) as claimed in any one of the preceding claims, **characterized in that** it comprises a hot air dryer (40) suitable for blowing a hot air flow into said dispenser (1) to dry said push bar (80) of the trolley.

## Patentansprüche

1. Vorrichtung (11) zur Hygienisierung von Einkaufswagen (8), umfassend einen Wagenkorpus und einen Schiebegriff (80),
wobei die Hygienisierungsvorrichtung (11) Folgendes umfasst:
- einen Tank (20) mit einem Hygienisierungsprodukt (L),
- einen Spender (1), der mit dem Tank (20) verbunden ist, um das Hygenisierungsprodukt abzugeben,
- Positionierungsmittel (4), um den Einkaufswagen (8) bezogen auf den Spender (1) zu positionieren; wobei die Positionierungsmittel (4) dazu geeignet sind, den Schiebegriff (80) des Einkaufswagens in einem Wirkbereich des Spenders (1) zu positionieren, um den Schiebegriff zu hygienisieren,
**dadurch gekennzeichnet, dass**
die Hygienisierungsvorrichtung (11) einen Empfänger/Sender (50) umfasst, der mit einem Transponder (51) gekoppelt ist, der auf dem Einkaufswagen (8) positioniert ist, um einen Zyklus zur Abgabe des Hygienisierungsprodukts zu aktivieren, wenn der Empfänger/Sender (50) die Anwesenheit des Transponders (51) des Einkaufswagens feststellt.

2. Vorrichtung (11) nach Anspruch 1, wobei der Einkaufswagen Vorderräder und Hinterräder umfasst und die Positionierungsmittel (4) eine konkave Führung (4) umfassen, die dazu geeignet ist, die Hinterräder des Einkaufswagens (8) in Eingriff zu nehmen.

3. Vorrichtung (11) nach Anspruch 1 oder 2, wobei der Spender (1) eine Spenderhülse umfasst, die sich über die Länge des Schiebegriffes (80) des Einkaufswagens erstreckt, wobei die Spenderhülse mit Düsen (10) zur Ausgabe des Hygienisierungsprodukts versehen ist, die entlang der Spenderhülse verteilt sind.

4. Vorrichtung (11) nach Anspruch 1, 2 oder 3, umfassend Mittel zur Betätigung des Spenders (1), die dazu geeignet sind, den Spender (1) aus einer ersten Warteposition, in der sich der Spender distal bezogen auf den Schiebegriff (80) des Einkaufswagens (8) befindet, in eine zweite Arbeitsposition zu verschieben, in der der Spender (1) den Schiebegriff (80) des Einkaufswagens (8) umschließt.

5. Vorrichtung (11) nach dem vorstehenden Anspruch, wobei der Spender (1) mindestens einen Hindernissensor umfasst, der dazu geeignet ist, die in einem Bewegungsraum des Spenders befindlichen Gegenstände zu erfassen und die Betätigung des Spenders (1) zu verhindern, wenn der Sensor ein Hindernis feststellt.

6. Vorrichtung (11) nach einem beliebigen der vorstehenden Ansprüche, umfassend eine Druckluftquelle, wobei der Spender (1) Düsen (9) zur Ausgabe von Druckluft umfasst, die mit der Druckluftquelle verbunden sind, um Luft in Richtung des Schiebegriffes (80) des Einkaufswagens (8) abzugeben.

7. Vorrichtung (11) nach einem beliebigen der Ansprüche von 2 bis 6, umfassend Mittel zur Bewegung der Führung (4), die dazu geeignet sind, eine Bewegung der Führung in mindestens eine vertikale Richtung zu bewirken.

8. Vorrichtung (11) nach einem oder mehreren der vorstehenden Ansprüche, umfassend ein Gehäuse (5), das mit dem Spender (1) gekoppelt und dazu bestimmt ist, mindestens eines der folgenden Elemente aufzunehmen: den Tank mit dem Hygienisierungsprodukt, die Druckluftquelle, die Mittel zur Betätigung des Spenders (1); wobei das Gehäuse (5) mindestens mit den Positionierungsmitteln (4) gekoppelt ist.

9. Vorrichtung (11) nach dem vorstehenden Anspruch, wobei das Gehäuse (5) einen manuellen Spender (7) für das Hygienisierungsprodukt umfasst.

10. Vorrichtung (11) nach einem beliebigen der vorstehenden Ansprüche, **dadurch gekennzeichnet, dass** der Tank (20) einen Zerstäuber (22) umfasst, um das Hygienisierungsprodukt (L) zu zerstäuben, sowie ein Lüfterrad (25), um das zerstäubte Hygienisierungsprodukt (G) in Richtung Spender (1) zu schieben.

11. Vorrichtung (11) nach Anspruch 10, **dadurch gekennzeichnet, dass** das Hygienisierungsprodukt eine Flüssigkeit (L) ist und der Zerstäuber (22) auf einem Schwimmer (21) angeordnet ist, der auf der Flüssigkeit (L) schwimmt.

12. Vorrichtung (11) nach Anspruch 10 oder 11, **dadurch gekennzeichnet, dass** der Spender eine Hülse (30) mit einem halbrunden oder halbachteckigen Querschnitt und Ableitbleche (31) umfasst, die unten in die Hülse (30) hineinragen, um das zerstäubte Gas (G) in Richtung des Schiebegriffes (80) des Einkaufswagens abzuleiten.

13. Vorrichtung (11) nach einem beliebigen der vorstehenden Ansprüche, **dadurch gekennzeichnet, dass** sie einen Heißlufttrockner (40) umfasst, der dazu geeignet ist, einen Heißluftstoß in den Zerstäuber (1) einzublasen, um den Schiebegriff (80) des Einkaufswagens zu trocknen.

## Revendications

1. Dispositif (11) d'assainissement pour chariots (11), ledit chariot (8) comprenant un corps de chariot et une barre de poussée (80),
ledit dispositif d'assainissement (11) comprenant :
- un réservoir (20) contenant un produit désinfectant (L),
- un ajutage (1) branché au dit réservoir (20) pour distribuer ledit produit désinfectant,
- des moyens de positionnement (4) pour positionner ledit chariot (8) par rapport au dit ajutage (1) ; lesdits moyens de positionnement (4) étant aptes à positionner ladite barre de poussée (80) du chariot en correspondance d'une zone active du dit ajutage (1) pour désinfecter ladite barre de poussée,
**caractérisé en ce que**
ledit dispositif d'assainissement (11) comprend un émetteur-récepteur (50) couplé à un transpondeur (51) positionné sur ledit chariot (8), dans le but d'activer un cycle de distribution du dit produit désinfectant lorsque l'émetteur-récepteur (50) capte la présence du transpondeur (51) du chariot.

2. Dispositif (11) selon la revendication 1, où ledit chariot comprend des roues antérieures et postérieures et lesdits moyens de positionnement (4) comprennent une coulisse (4) concave apte à engager lesdites roues postérieures du dit chariot (8).

3. Dispositif (11) selon les revendications 1 ou 2, où ledit ajutage (1) comprend un manchon de distribution qui se déploie pour la longueur de la barre de poussée (80) du chariot, ledit manchon de distribution étant muni de buses (10) de distribution du dit produit désinfectant réparties le long du dit manchon de distribution.

4. Dispositif (11) selon les revendications 1, 2 ou 3, comprenant des moyens d'activation du dit ajutage (1) aptes à déplacer ledit ajutage (1) entre une première position d'attente, distante par rapport à ladite barre de poussée (80) du dit chariot et une seconde position de fonctionnement où ledit ajutage (1) entoure ladite barre de poussée (80) du chariot (8).

5. Dispositif (11) selon la revendication précédente, où ledit ajutage (1) comprend au moins un capteur d'obstacles apte à détecter la présence d'objets dans l'espace de mouvement du dit ajutage et empêcher l'activation du dit ajutage (1) lorsque ledit capteur détecte un obstacle.

6. Dispositif (11) selon l'une quelconque des revendications précédentes, comprenant une source d'air comprimé et où ledit ajutage (1) comprend des buses (9) de distribution d'air comprimé branchées à ladite source d'air comprimé pour la distribution d'air en direction de ladite barre de poussée (80) du chariot (8).

7. Dispositif (11) selon l'une quelconque des revendications de 2 à 6, comprenant des moyens de déplacement de ladite coulisse (4) aptes à déterminer un déplacement de ladite coulisse dans au moins une direction verticale.

8. Dispositif (11) selon l'une ou plusieurs des revendications précédentes, comprenant un caisson de logement (5) couplé au dit ajutage (1) et destiné à contenir au moins l'un des éléments suivants : ledit réservoir d'un produit désinfectant, ladite source d'air comprimé, lesdits moyens d'activation du dit ajutage (1) et où ledit caisson (5) est au moins couplé aux dits moyens de positionnement (4).

9. Dispositif (11) selon la revendication précédente, où ledit caisson (5) comprend un distributeur manuel (7) de produit désinfectant.

10. Dispositif (11) selon l'une quelconque des revendications précédentes, **caractérisé en ce que** ledit réservoir (20) comprend un nébuliseur (22) pour nébuliser ledit produit désinfectant (L) et un ventilateur (25) pour pousser ledit produit désinfectant nébulisé (G) vers ledit ajutage (1).

11. Dispositif (11) selon la revendication 10, **caractérisé en ce que** ledit produit désinfectant est un liquide (L) et ledit nébuliseur (22) est disposé dans un flotteur (21) qui flotte sur ledit liquide (L).

12. Dispositif (11) selon les revendications 10 ou 11, **caractérisé en ce que** ledit ajutage comprend un manchon (30) ayant une section transversale de forme semi-circulaire ou semi-octogonale et des ailettes de déflection (31) qui font saillie inférieurement dans ledit manchon (30) pour convoyer ledit gaz nébulisé (G) vers ladite barre de poussée (80) du chariot.

13. Dispositif (11) selon l'une quelconque des revendications précédentes, **caractérisé en ce qu'**il comprend un sécheur (40) à jet d'air chaud pour introduire un jet d'air chaud dans ledit ajutage (1) pour sécher ladite barre de poussée (80) du chariot.
